# EUROPEAN PATENT APPLICATION

(11) **EP 1 586 875 A1**
(43) Date of publication of application: **19.10.2005**
(21) Application number: 05270008.5
(22) Date of filing: 08.04.2005
(51) Int. Cl.: G01H 1/00

(54) **Vibrational dosimeter**

(30) Priority: 16.04.2004 GB 0408493
(71) Applicant: Mecon Limited, Histon, Cambridgeshire CB4 9ES (GB)
(72) Inventor: Thompson, Martin, CB4 9ES, Cambridge (GB); Harper, Mark, CB4 3QH, Cambridge (GB)
(74) Representative: Tolfree, Roger

(57) **Abstract**

Regulating the exposure of potentially damaging hand-arm vibrations to machine operators requires cheap, easy-to-use personalised devices which will measure and record the cumulative exposure of vibrations to an operator during the course of a working period. The invention provides a dosimeter (1) adapted to fit onto an operator's hand comprising a sensor (19), to measure vibration levels to which the operator is exposed, a means (14) to calculate the cumulative exposure and a means (12) to communicate the collected data to the operator.

## Description

The present invention relates to a method and apparatus for measuring the exposure of a machine operator to potentially harmful vibration.

Currently there are no cheap and easy-to-use personalized devices available to measure hand-arm vibration on a regular basis. Yet there is EU legislation now in place that requires the monitoring of such vibration on an increasing basis from 6^{th} July 2005 until complete implementation five years later. This invention arose when considering how it would be possible to provide the necessary means of monitoring that will satisfy the requirements of the legislation while at the same time satisfying the industry requirements of being robust, cheap and simple to use.

The new Directive acknowledges the possible damaging consequences of vibration for human health and lays down maximum levels of vibration exposure to avoid "white finger". White finger is a medical condition of numbness or pain that arises from continuous use of vibrating tools. In extreme cases white finger can lead to loss of one or more digits. The new Directive lays down a careful specification of what cumulative vibration levels the user is allowed to be exposed to. Above this specified dosage work must stop for that day. What the Directive does not do is suggest how this exposure is to be measured. Technically vibration measurements can be made with high quality expensive hardware that exists on the market today. This hardware will have been used in research work involved in the definition of the standards. However the Directive requires an all-embracing assessment of vibration levels for workers that cannot be carried out today without expensive equipment.

Up to now approaches to the problem have mainly involved attempts to classify particular vibration prone tools such as drills and sanders in terms of their expected vibration level. An example of this approach is a dosage meter supplied by Castle Group Ltd of Scarborough. A hand held device measures the acceleration of the tool handle producing a signal in a cable that is routed to a processing and display unit worn on a belt. Using a single tool, the user can use a simple calculation from period of use to work out the dosage. Using multiple tools involves timing the period of use of each one and entering the figures into a spreadsheet that calculates the total dosage. There are numerous difficulties with this approach. Tools cannot be accurately classified by type because vibration levels will vary with age and condition of the machine, with weight of the user, with pressure applied to the grip and with the experience of the user. Some working gloves are designed to mitigate the vibration felt by the user and measuring on the machine will not allow for this mitigation. Further, when using multiple tools it is not easy for the user to keep track of the duration of use of each one. Entering the data into a spreadsheet is not convenient and may be too late - the dosage limit may have already been exceeded.

There is a need to provide a vibration dosage meter that is light, unobtrusive, comfortable and easy to use even when wearing work gloves and cheap enough for every worker to have one. It must calculate the vibration dose accurately and provide a clear indication of when the dosage limit has been reached. This invention arose in an endeavour to meet these requirements.

According to the invention there is provided a method of controlling the exposure of a machine operator to potentially harmful vibration characterised in comprising: positioning a sensor housing on the operator's hand; deriving, within the housing, a measurement of vibration; calculating, also within the housing, an assessment of the operator's cumulative exposure to the vibration; and indicating the cumulative exposure to the operator so that the operator is alerted before such exposure exceeds a maximum safe value.

The invention also provides apparatus for measuring the exposure of a machine operator to potentially harmful vibration characterised in comprising: a sensor unit adapted to be held on the hand of the operator so as to sense vibration at a point between the operator's hand and a handle of the machine; and means, carried by the sensor unit, for monitoring an output of the sensor and to produce an assessment of the operator's cumulative exposure to the vibration.

The sensor unit is preferably a self contained cufflink shaped device sized such that the link or neck section fits between any pair of fingers of the hand while the base of the device sits on the underside of the hand and is shaped so as to ensure that when the hand grips a handle the base presses against that handle and a display on the device itself indicates when the allowed level of cumulative dosage of vibration input to the fingers has been reached. The top of the device preferably sits across the pair of fingers and is sized to be comfortable when worn inside a work glove. The top of the dosimeter preferably contains a triaxial accelerometer, means of conditioning the accelerometer signals, some means of filtering, digitising and processing the signals to give a cumulative measure of vibration dosage in accordance with the aforesaid EU Directive, data storage, a clock, a small battery to power the unit and means of displaying the results. In this arrangement the base of the unit and the link or neck serve to hold the sensor in position on the operator's hand and also to transmit vibration from a point between the operator's hand and the handle of the tool to the top of the unit where the accelerometer is located.

The processor in the top of the sensor may be designed to calculate other simple measures or parameters of vibration such as mean level and duration. Means may be provided of downloading the data to an external storage medium such as a personal computer. No external connections are required during use of the device. The battery may be disposable or preferably rechargeable. The means of recharging may be by cable from an external charger or the device may have a built in antenna to allow non-contact recharging. Recharging will only be necessary once daily. The means of downloading data will be by means of a wire connection, infrared or wireless. The device will incorporate sufficient storage for data from a full working day of at least eight hours.

The "cuff-link" shaped configuration described above is believed to be particularly convenient and comfortable to use, and is considered to be inventive itself. Accordingly, another aspect of the invention provides a vibration dosage meter characterised in comprising a sensor unit having a neck which fits between the fingers of an operator and enlarged portions at either end of the neck, which, in use, lie on the front and back of the hand serving to hold the sensor unit in a position to sense vibration from a handle of a machine held by the operator.

It is proposed that, at the start of the working day each user will collect their own fully charged vibration dose meter, check that the dosimeter is active and then wear it for the remainder of the day. Working gloves, if worn, shall go on top of the dosimeter. Bulky gloves can act as a barrier to vibration and some are explicitly designed to do so but the dosimeter measures within the glove and records the vibration level input to the fingers. A display on top of the dosimeter will give a clear indication of the dosage that has been received at any time. If the maximum allowed dosage level has been reached then the working day should end. At the end of a working day the dosimeter may be returned to a docking station where it may be recharged and the data may be downloaded. The additional data that are downloaded will allow detection of any attempts to cheat the system by building up a dosage figure without wearing the device, for example by strapping it to a constantly vibrating machine. Similarly the parameters will allow ready detection of any attempt to cheat the system by not wearing the device.

A specific embodiment of the invention is now described by way of example with reference to the accompanying drawings in which: -
Figure 1A shows a plan view of a dosimeter constructed in accordance with the invention;
Figure 1B is a side elevation of the dosimeter of Fig 1A
Figure 1C is an end elevation of the dosimeter of Fig 1A
Figure 1D is a bottom view of the dosimeter of Fig 1A
Figure 2 shows the dosimeter in use from the underside without a glove;
Figure 3 shows the dosimeter in use from the top without a glove;
Figures 4A, 4B and 4C show schematically the contents of the dosimeter;
Figure 5 shows schematically the dosimeter connected to a docking station for data download; and
Figure 6 shows schematically the dosimeter in use on a vibrating handle.

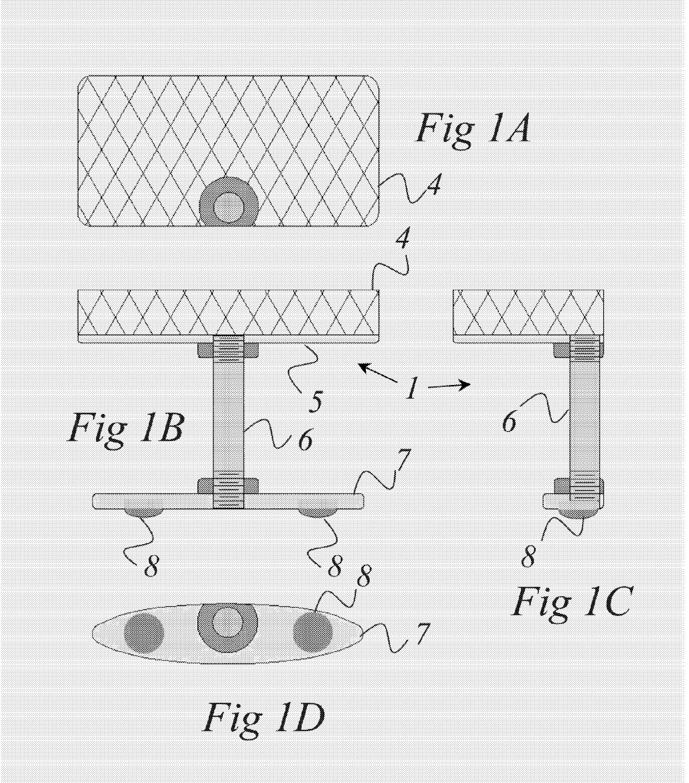

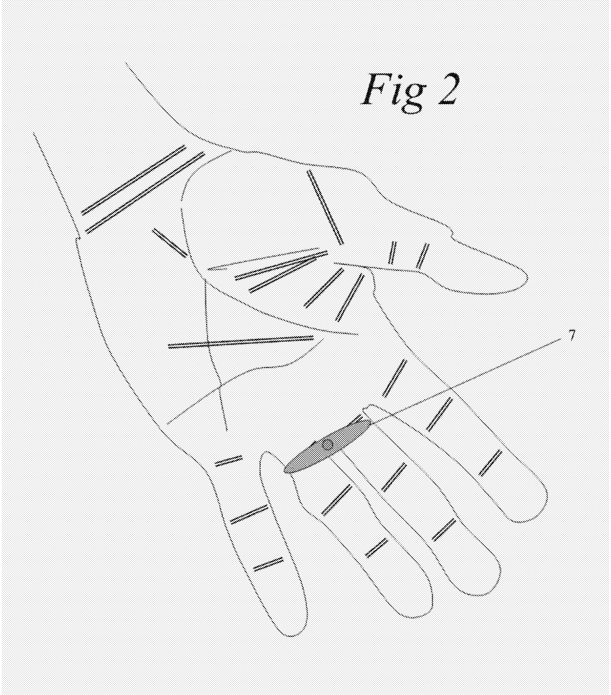

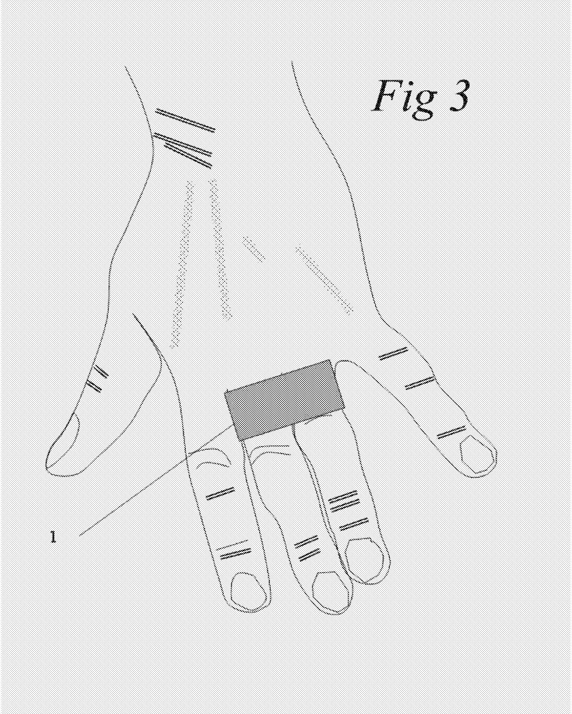

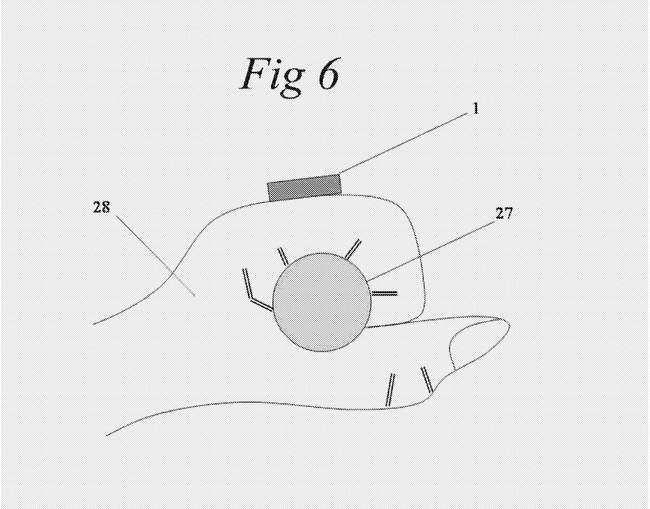

Referring to Figs 1A to 1D, there is shown a container 4 for an LCD display and electronics. The sensor, electronics, display and battery are within the container 4. The container 4 has a support 5 for strength. The finger grip 6 joins the support 5 that sits above the fingers with the contact bar 7 that sits between the fingers and the surface that is being gripped. Below the contact bar 7 are two contact buttons 8 that ensure good contact with the vibrating surface. A suitable material for manufacture of the container support, finger grip and contact bar could be ABS plastic to minimize corrosive action and minimize transient impact force. The electronics is designed to be low power with no moving parts. A suitable means of protecting the electronics would be to embed them in a suitable epoxy. The protective means of embedding may be clear to allow it to encapsulate the LCD display and soft to avoid impact damage. Normally a working glove will be worn over the hand and the dosimeter. This glove is not shown on the schematic drawings. In figure 2 is shown the dosimeter 1 from the underside when worn on the hand. In figure 3 is shown the dosimeter 1 from the top when worn on the hand. In figure 6 is shown the dosimeter 1 when worn on a hand 28 gripping a vibrating tool 27.

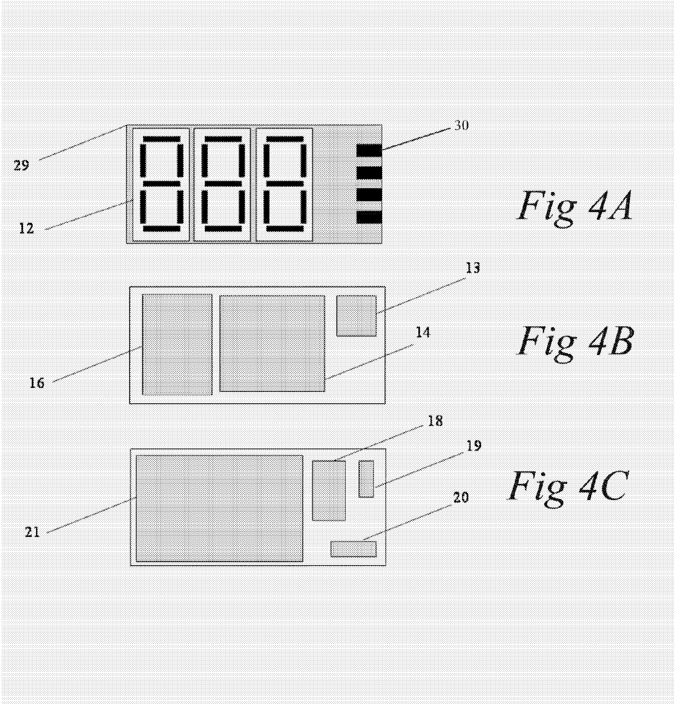

Figures 4A to 4C show three views of the dosimeter from above. The first view 4A shows the top view of the dosimeter with three liquid crystal alphanumeric elements (LCD) 12 as a display. A suitable display would be the Densitron DG729 3 digit 7 segment LCD. At the edge of the top of the dosimeter are electrical contacts 30. Data download and battery recharging is by means of these contacts 30 together with the electrically screened underside. The second view 4B shows the dosimeter from above with the LCD elements removed to expose the top of the electronics layer. The processor 14 sits between the non-volatile memory (EEPROM) 13 and the battery management and external serial link (RS232) 16. A suitable processor would be the Texas Instruments MSP340F447. A suitable EEPROM would be the Atmel AT250080A. The processor 14 contains within it the analogue to digital converter (ADC). The arrangement of these items within the electronics layer is not of any importance. The processor 14 may implement the shaped filter required by ISO standard 5349-1(2001) and calculate the running vibration dose according to the algorithm given in that ISO standard. The vibration dose will be displayed on the LCD elements in a clear unambiguous manner. Other statistics of the vibration exposure will be calculated as required and all parameters will be stored in the non-volatile memory EEPROM 13. These parameters will be optionally downloaded to an external PC by means of the external RS232 link. When downloaded successfully the EEPROM will be erased ready for reuse. The RS232 link 16 also supplies the recharging power for the battery 21. A suitable device to control the charging would be the Maxim MAX1736. A suitable device for the RS232 level translator interface would be the Maxim MAX3226E. The third view 4C shows the underside of the electronics layer. The battery 21 takes up most of the space and this sits along side the three direction of motion acceleration sensor 19. A suitable battery would be a lithium rechargeable unit. The motion sensor 19 is shown as a single device but could be in the form of two devices. A suitable single device sensor would be a planar three-axis micro-electronics mechanical sensor (MEMS) from European Technology for Business Ltd (ETB). The anti-alias filter 18 is shown as a separate device although this function can be incorporated within the processor 14 as long as the ADC within the processor 14 is fast enough. Suitable anti-alias filters would be based around the MAX4163 operational amplifier.

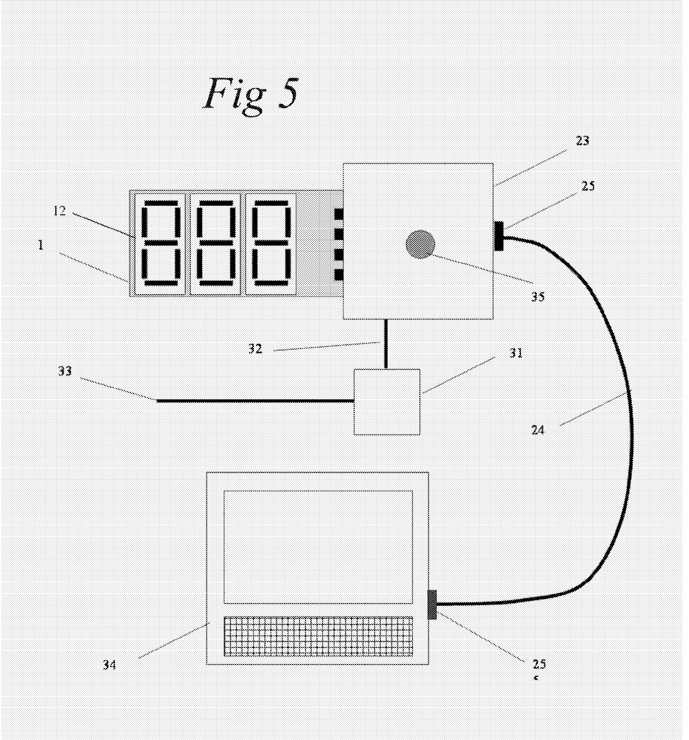

In figure 5 is shown the dosimeter 1 plugged into the docking station 23. The docking station 23 can be used solely for recharging in which case it is supplied with a suitable low voltage by cable 32 from a charger 31 connected by cable 33 to the mains power. To obtain download of the data from the dosimeter 1 the docking station 23 is connected 25 by Universal Serial Bus (USB) cable 24 to a personal computer (PC) 34. The conversion from USB protocol from the PC 34 to RS232 protocol on the dosimeter is carried out in the docking station 23. On the docking station 23 is a button 35. When this button 35 is pressed while the dosimeter 1 is withdrawn from the docking station 23 then the dosimeter is left in a very low current inactive mode suitable for long-term storage. If the button 35 is not pressed while the dosimeter 1 is withdrawn then the dosimeter 1 will be active and the LCD display 12 will indicate this.

Numerous variations to the illustrated system are possible within the scope of the invention. For example the cuff-link shape of the sensor, whilst very effective, is not essential. Instead, a sensor and possibly also the display could be incorporated into the structure of a glove or a ring or band that fits over one finger. Also, although a display is desirable, it would be adequate to provide a simple warning signal when the designated exposure level is reached. The illustrated system is however believed to provide one particularly simple and effective way of ensuring that safe levels of exposure to vibration are not exceeded

## Claims

1. A method of controlling the exposure of a machine operator to potentially harmful vibration **characterised by** the steps of: positioning a sensor housing on the operator's hand; deriving, within the housing, a measurement of vibration; calculating, also within the housing, an assessment of the operator's cumulative exposure to the vibration; and indicating the cumulative exposure to the operator so that the operator is alerted before such exposure exceeds a maximum safe value.

2. A method according to Claim 1 **characterised in that** the vibration is sensed at a point between the operator's hand and a handle of the tool.

3. A method according to Claim 1 or 2 **characterised in that** the output of the sensor is a measurement of the severity of the vibration and in which this output is used to derive the assessment of cumulative exposure.

4. Apparatus for measuring the exposure of a machine operator to potentially harmful vibration **characterised by**: a sensor unit (1) adapted to be held on the hand of the operator so as to sense vibration at a point between the operator's hand (28) and a handle of the machine (27); and means, carried by the sensor unit (1), for monitoring an output of the sensor (19) and to produce an assessment of the operator's cumulative exposure to the vibration.

5. Apparatus according to Claim 8 **characterised in that** the sensor unit (19) is adapted to sense vibration at the surface of the operator's hand (28).

6. Apparatus according to Claim 8 or 9 **characterised in that** the sensor (19) is designed to give an output which is a measurement of the severity of vibration and in which the sensor unit (1) includes a clock and means for calculating from the outputs of the clock and the sensor the assessment of cumulative exposure.

7. Apparatus according to Claim 8, 9 or 10 **characterised by** an indicator (12), carried by the sensor unit, arranged to indicate to the operator when cumulative exposure has exceeded a safety limit.

8. Apparatus according to any one of Claims 8 to 11 **characterised in that** the sensor unit (1) is designed to transmit vibration from the palm-side of the hand, along a link to a sensor on the back of the hand.

9. Apparatus according to any one of Claims 8 to 12 **characterised in that** the sensor unit (1) has: a neck (6) which fits in use between the operator's fingers, the neck joining two relatively wide end portions, one of the end portions (4,5) housing an accelerometer and being designed to lie against the back of the operator's hand, whilst the other end portion (7) lies against the palm-side of the hand so that vibration is transmitted from a handle of a vibrating machine, along the neck to the accelerometer.

10. Apparatus according to any one of Claims 8 to 13 **characterised by** a docking station (23) designed to receive the sensor unit (1); the sensor unit (1) and the docking station (23) having corresponding contacts allowing data to be downloaded from the sensor unit (1) to the docking station (23) or through the docking station to a separate computer (34).

11. A vibration dosage meter **characterised by** a sensor unit (1) having a neck (6) which fits between the fingers of an operator and enlarged portions at either end of the neck, which, in use, lie on the front and back of the hand serving to hold the sensor unit (1) in a position to sense vibration from a handle of a machine held by the operator.
